Europäisches Patentamt

European Patent Office

Office européen des brevets

(11) Publication number: **0 106 495**
A2

# (12) EUROPEAN PATENT APPLICATION

(21) Application number: **83305183.2**

(51) Int. Cl.³: **G 01 N 33/54**

(22) Date of filing: **06.09.83**

(30) Priority: **17.09.82 JP 160886/82**

(43) Date of publication of application:
**25.04.84 Bulletin 84/17**

(84) Designated Contracting States:
**CH DE FR GB IT LI NL**

(71) Applicant: **FUJIREBIO KABUSHIKI KAISHA also trading as FUJIREBIO INC.**
**6-7, Shimoochiai 4-chome Shinjuku-ku**
**Tokyo 161(JP)**

(72) Inventor: **Ikeda, Mikio**
**7-25-23, Sunagawacho**
**Tachikawa-shi Tokyo(JP)**

(72) Inventor: **Nakamizo, Yasuzo**

**Deceased(JP)**

(74) Representative: **Silverman, Warren et al,**
**HASELTINE LAKE & CO. Hazlitt House 28 Southampton Buildings Chancery Lane**
**London WC2A 1AT(GB)**

(54) Method and reagent for detecting antivirus antibody.

(57) A reagent for detecting antivirus antibody comprises carrier particles which do not aggregate to any significant extent in the presence of a virus having hemagglutinating activity, and a viral antigen sensitized on the carrier particles. The reagent is used to detect antivirus antibody by the indirect passive hemagglutination technique.

EP 0 106 495 A2

"METHOD AND REAGENT FOR DETECTING ANTIVIRUS ANTIBODY"

This invention relates to a method of detecting antivirus antibody and to a reagent for use therein.

Neutralisation, hemagglutination inhibition and complement fixation tests have been developed for use in detecting viral infectious diseases, such as rubella, measles, mumps, and parainfluenza. However, the neutralisation test requires a long time to carry out and special operating techniques. Conditions for the hemagglutination inhibition test need to be adjusted to take account of differences between antigen and blood cell characters of different subjects. Indeed with the latter test, the fact that the antigen is unstable and that it is complicated to carry out is also a problem. The complement fixation method is also not satisfactory in requiring a long measuring period and precision in its carrying out. Additional to the above methods are antibody detection methods using immune reaction, as exemplified by indirect passive hemagglutination. However, this method is not suitable for detecting antivirus antibody, because most viruses themselves have hemagglutinating activity.

It is an object of this invention to provide a method of detecting antivirus antibody using indirect passive hemagglutination which is not subject to the aforementioned disadvantage.

According to one aspect of this invention, there is provided a reagent for use in the detection of antivirus antibody which comprises carrier particles which have the property of not aggregating to any significant extent in the presence of a virus having hemagglutinating activity and which have a particle diameter in the range of from 0.5 to 20 $\mu$m and a viral antigen having hemagglutinating activity and which is sensitized on the carrier particles.

According to a second aspect of this invention, there is provided a method of detecting antivirus antibody, which comprises suspending in a liquid suspension medium the sensitized particles of the reagent of the invention, mixing a dilution series of a test sample with said suspension and identifying the existence of the antibody in any test mixture thus produced by the agglutination of sensitized particles therein.

It has now been found that certain carrier particles do not aggregate in the presence of a virus having hemagglutinating activity, and that the antibody of this virus can then easily and reliably be detected by indirect passive particle-agglutination by employing such carrier particles.

It is a feature of such carrier particles that they do not aggregate to any significant extent in the presence of a virus having hemagglutinating activity. In order to achieve a suitable agglutination pattern, it is necessary for the particles to have a diameter of from 0.5 to 20 μm, preferably from 1 to 10 μm. The particles will be insoluble in the liquid medium in which indirect passive hemagglutination is carried out. The particles will usually be spherical and may be homogeneous with inner parts of the particles being constituted by the same material as their surface, or the particles may have inner parts of different constitution to surface material, i.e. the particles may be formed as microcapsules. In order to facilitate judgement of an agglutination pattern, the particles are preferably coloured. In addition to possessing the above properties, it is necessary that the carrier particles be capable of being sensitized with a viral antigen. The sensitisation will be described later.

Specific examples of carrier particles which may be used are gelatin particles, such as described in EP-A-O 062 968, polyacrylamide particles such as Affi-Gel 701, Affi-Gel 702 and Immunobead Reagent (made by Bio-Rad Laboratories), microcapsules, for example those made from polyurethane, microbial cells such as serratia, polystyrene latex, and carbon powder. Gelatin particles and polyacrylamide particles are the most preferable because of the minimal nonspecific agglutination which they show.

Gelatin particles according to EP-A-0 062 962 are produced from a gelatin solution containing a water-soluble polysaccharide and a metaphosphate. Any commercially available gelatin material may be used, although gelatines having an isoelectric point at a pH of from 8 to 9 are preferable. The water-soluble polysaccharides used are those which are conventionally employed as viscosity increasing agents, being possibly used in the form of a paste, and include derivatives and salts of such polysaccharides. Examples of such polysaccharides are gum arabic, carboxymethyl cellulose, sodium alginate and agar. The metaphosphate is a compound having the formula of $(MPO_3)_n$, where M is cation and n is a whole number from 3 to 6, and is exemplified by sodium trimetaphosphate and sodium hexametaphosphate.

The concentration of gelatin in the solution is usually from 0.01 to 2%, preferably from 0.05 to 1.0% by weight, the concentration of the water-soluble polysaccharide is usually from 0.01 to 2%, preferably from 0.05 to 1.0% by weight, and the concentration of metaphosphate is usually from 3 to 15% by dry weight of the gelatin. The concentration of each component selected in practice will depend on the desired size of particles and other physical properties of the particles, such as hardness.

The solution may also contain such other components as a water-miscible organic solvent, a nonionic or anionic surfactant, and a colouring agent.

The temperature of the solution from which the particles are to form should be higher than the gelation temperature of the gelatin. The gelation temperture depends _inter_ _alia_ on the concentration of the gelatin, and it is usually in the range of from 25 to 35°C. The temperature of the solution is preferably in the range of from 35 to 50°C for optimisation of particle formation.

The pH of the solution from which particles are to be formed is adjusted to 2.5 to 6.0 by adding acid while the solution is stirred. During the pH adjustment, particles are produced in the solution. In order to produce uniform particles, the acid is added dropwise to the solution while heating it at from 35 to 50°C and subjecting it to gentle stirring. The optimal pH within the range of pH 2.5 to 6.0 depends on the desired size of particles and the composition of the solution. In order to produce particles sized in the range of from 0.5 to 20 μm the pH is preferably adjusted to be from 4.0 to 5.5. The character of the acid employed for the pH adjustment is not critical, and either an inorganic acid or organic acid may be employed. However, a weak acid, for example acetic acid, is preferred.

After the addition of the acid, the suspension of the particles which have formed is immediately cooled below 10°C in order to prevent aggregation of the particles. Then, an aldehyde cross-linking agent is added to the suspension, and the particles are insolubilised by having them stand overnight at a temperature of not more than 10°C. The amount of cross-linking agent used is from 0.1 to 200% of the dry weight of gelatin. The cross-linking agent is preferably selected from glutaraldehyde, formaldehyde, glyoxal, crotonaldehyde, acrolein, and acetaldehyde. Glutaraldehyde is the most preferred.

After treatment with the cross-linking agent, the particles are recovered by means of for example centrifugation, and usually washed twice or three times with water containing surfactant.

The particles thus produced sometimes swell in a salt solution, and accordingly, it is preferable that the particles be further treated with formaldehyde.

The viral antigen used in the reagent of this invention has hemagglutinating activity. Most viruses have hemagglutinating activity, and examples of such viruses are rubella virus, measles virus, mumps virus, parainfluenza virus type-1 and parainfluenza virus type-2. Any commercially available viral antigen of such a virus may be used. Alternatively, the viral antigen may be prepared specifically for the reagent using an appropriate method.

Sensitisation of the carrier particles with the viral antigen may be carried out by chemical reaction or by adsorption. However, it is most preferable that the viral antigen be covalently bonded to the carrier particles.

When the viral antigen is covalently bonded to the carrier particles, the sensitisation may be carried out by means of a conventional sensitisation procedure using mammalian erythrocytes as the carrier. Such sensitisation method includes procedures using any one of tannic acid, formalin, glutaraldehyde, the pyruvic aldehyde, bis-diazotized benzidine and toluene-2,4-diisocyanate. The sensitisation may also be carried out by the immobilistion method for enzyme. Typically this method involves the formation of covalent bonds utilising for this purpose amino groups, carboxyl groups or hydroxyl groups of the viral antigen. The immobilisation method also includes the diazotization method and the acid azide method. Of these various sensitisation methods, the sensitisation methods for erythrocytes are preferable, and in the case of the gelatin particles, the tannic acid method and the glutaraldehyde method are the most preferable. When the carrier particles are polyacrylamide beads, the use of a carbodiimide regent such as 1-ethyl-3[3-dimethylaminopropyl]carbodiimide.HCl for the sensitization is preferred.

The viral antigen sensitized carrier particles thus produced are usually lyophilised before being used as the reagent for detecting antivirus antibody. The lyophilised matter is preferably preserved at a low temperature such as at 4°C. This viral antigen sensitized carrier will generally be combined with an adjuvant which is for example a diluent, a standard serum, an unsensitized carrier, a reconstituting solution or an absorbing solution. For example, kaolin or heparin-$MnCl_2$ may be used as absorbing solution for the rubella virus antigen, and $KIO_4$ or trypsin may be used for the mumps virus antigen.

The detection of antivirus antibody using the reagent of this invention may be carried out by employing otherwise conventional procedures for detecting antibody using indirect passive hemagglutination. Namely, the viral antigen sensitized carrier particles are first suspended in a liquid medium in a suitable concentration, and the suspension is mixed with a dilution series of a test sample. The agglutination of the sensitized particles which takes place for each mixture is noted. The suspension of the sensitized particles is usually carried out by using a reconstituting solution and/or diluent. The sample can be one of a variety of materials, but it is usually a body fluid such as serum. The dilution series, for example a twice by twice dilution series or four times by four times dilution series, is prepared using the diluent. The detection is, for example, carried out according to the micro titre method. The existence of agglutination of the sensitized particles is usually decided by observation with the naked eye. However, it may also be decided mechanically.

By appropriately selecting the antigen to be sensitized, it is possible to produce a reagent of the present invention able to detect every antivirus antibody. Accordingly, various viral infectious diseases can easily and reliably be detected in a short time by using such a reagent. The reagent of the invention is stable. For example, in the case of rubella virus antigen, the conventionally used reagent must be used within 30 minutes after reconstitution, whereas a reagent according to the invention produced by using gelatin particles can still be used for about two weeks when it is preserved at 4°C after reconstitution.

The present invention is further illustrated by the following examples. In the examples percentages are expressed on a weight basis unless otherwise indicated.

EXAMPLE 1

(1) Preparation of Gelatin Particles

4 grams of gelatin having an isoelectric point at a pH of 9 were dissolved in water at 40°C, and the volume of the solution obtained was adjusted to 100ml. The pH of the solution was then adjusted to 9. 4 grams of gum arabic were dissolved in water, and the volume of the solution was also adjusted to 100 ml. Insoluble matter was filtered off, and the filtrate obtained was warmed to 40°C.

50 ml of the gelatin solution and 50 ml of the filtrate were mixed, and the mixture produced was poured into 300 ml of 30 vol% aqueous methanol which had previously been warmed to 40°C. The mixture was sufficiently stirred to achieve homogeneity.

1.6 ml of 10% sodium hexametaphosphate solution, 2 ml of 1% alkylsulphomaleic acid (trade name: Demol Ep, manufactured by Kao Soap Co.) and 6 ml of a 1% reactive blue solution were added to the mixture, and the mixed solution was sufficiently stirred. Subsequently, 10 vol% of acetic acid were added dropwise to the mixed solution while it was kept at 40°C. The pH of the mixed solution was adjusted to 4.8 and gelatine particles precipitated.

The suspension of particles was cooled to 5°C in an ice bath, and 1.3 g of glutaraldehyde was added to the suspension. After stirring, the suspension was allowed to stand at 5°C overnight. Then, the suspension was centrifuged at 2000 rpm for 10 minutes, and the resulting insolublised particles were collected. The particles were suspended in 0.005% Demol Ep solution, and then centrifuged again. The washing procedure was repeated three times, and the particles were suspended in 4 vol% of formalin. The suspension was then allowed to stand at 5°C for one week.

The yield of carrier particles was 7.7 g, and 75% of the particles were in the range of 3 to 6 um.

(2)  Preparation of Sensitized Particles

Vero cells were cultured, and rubella virus was separated from the culture liquid in the conventional manner. The rubella virus was suspended in a phosphate buffered saline (hereinafter referred to as PBS) of pH 7.2 in a concentration of 32 HA units, and the suspension was employed as a viral antigen.

The carrier particles produced as described under (1) were suspended in PBS of pH 7.2 containing 5 ppm tannic acid so that the concentration of the particles was 2.5%. The suspension was warmed at 37°C for 10 minutes. The particles were collected by centrifuging, and washed 4 times with a saline solution by means of centrifugation. The washed particles were suspended in PBS of pH 7.2 so that the concentration of the particles was 5%.

The suspension of the viral antigen was mixed with an equal volume of the suspension of the tannic acid-treated particles, and the mixture obtained was warmed at 37°C for 40 minutes to obtain thereby particles sensitized with the viral antigen. The sensitized particles were collected by centrifuging, and washed 4 times with a saline solution by centrifugation. The washed particles were then suspended in a dispersing medium so that the concentration of the particles was 5%. This suspension was rapidly frozen using liquid nitrogen, and dried in a lyophilising apparatus.

(3)  Detection of Antivirus Antibody

The particles sensitized with rubella virus antigen thus obtained were reconstituted using distilled water, and reacted with sera of rubella patients by the micro titre method. The results obtained are shown in Table 1. Antibody titres of these sera were measured by the conventional HI method by using "SERODIA-RUBELLA" (trade name, manufactured by Fujirebio Inc.), and the results obtained are also shown in Table 1. the results obtained with the method of the invention were well in accord with the results of the HI method.

T a b l e 1

Dilution Ratio of Serum

| Serum | 4 | 8 | 16 | 32 | 64 | 128 | 256 | 512 | HI antibody titre |
|---|---|---|---|---|---|---|---|---|---|
| 1 | ++ | + | ± | − | − | − | − | − | 16 |
| 2 | ++ | ++ | + | ± | − | − | − | − | 32 |
| 3 | ++ | ++ | ++ | + | ± | − | − | − | 64 |
| 4 | ++ | ++ | ++ | ++ | + | ± | − | − | 128 |
| 5 | ++ | ++ | ++ | ++ | + | ± | − | − | 256 |

Moreover, the reconstituted suspension was diluted with PBS of pH 7.2 so that the concentration of the particles was 1%. The diluted suspension was preserved at $4^\circ C$ for 2 weeks. In addition, particles were sealed in a vessel, and allowed to stand in an oven at $40^\circ C$ for 2 weeks. The titres of particles treated by each procedure were measured, and the results which are shown in Table 2 indicate the stability of the reconstituted particles at least two weeks after reconstitution.

T a b l e 2

Dilution Ratio of Serum

| | 4 | 8 | 16 | 32 | 64 | 128 | 256 | 512 |
|---|---|---|---|---|---|---|---|---|
| Immediately after reconstitution | ++ | ++ | ++ | ++ | ++ | + | − | − |
| 2 weeks after reconstitution | ++ | ++ | ++ | ++ | ++ | + | − | − |
| After 2 weeks at $40^\circ C$ | ++ | ++ | ++ | ++ | ++ | + | − | − |

EXAMPLE 2

(1)  Preparation of Sensitized Particles

Carrier particles produced in step (1) of Example 1 were suspended in PBS of pH 7.2 containing 1 ppm, 5 ppm or 10 ppm tannic acid so that the concentration of the particles was 2.5% in each case and each suspension was warmed at 37°C for 10 minutes.  The particles were separately collected by centrifugation from each suspension, and washed 4 times with a saline solution using centrifugation.  Each sample of washed particles was suspended in PBS of pH 7.2 so that the concentration of the particles was 5%.

A lyophilised HA antigen of measles virus (titre 1:32, manufactured by Denka Seiken Co.Ltd., Japan) was reconstituted with 1 ml of distilled water, and diluted twice by twice with PBS of pH 7.2 to prepare a dilution series of the viral antigen solution.

Each of the above suspensions was mixed with an equal volume of the above dilution series of the viral antigen solution, and each mixture was warmed at 37°C for 40 minutes to sensitize the particles.  Each sample of sensitized particles was washed and lyophilised in the same manner as Example 1.

(2)  Detection of Antivirus Antibody

The particles sensitized with measles virus antigen thus obtained were reacted with anti-measles virus anti-serum (titre 1:128, manufactured by Denka Seiken Co. Ltd., Japan) by the micro titre method.  The results obtained are shown in Table 3.

Table 3

| Tannic Acid | Dilution Ratio of Antigen | Dilution Ratio of Serum | | | | | | | |
|---|---|---|---|---|---|---|---|---|---|
| | | 4 | 8 | 16 | 32 | 64 | 128 | 256 | 512 |
| 1 | 8 | ++ | ++ | ++ | ++ | ++ | + | ± | - |
| | 16 | ++ | ++ | ++ | ++ | + | ± | - | - |
| | 32 | ++ | ++ | ++ | + | ± | - | - | - |
| | 64 | ++ | ++ | + | ± | - | - | - | - |
| | 128 | ++ | + | ± | - | - | - | - | - |
| 5 | 8 | ++ | ++ | ++ | ++ | ++ | ++ | + | ± |
| | 16 | ++ | ++ | ++ | ++ | ++ | + | ± | - |
| | 32 | ++ | ++ | ++ | ++ | + | ± | - | - |
| | 64 | ++ | ++ | ++ | + | ± | - | - | - |
| | 128 | ++ | ++ | ++ | + | ± | - | - | - |
| 10 | 8 | ++ | ++ | ++ | ++ | ++ | + | ± | - |
| | 16 | ++ | ++ | ++ | ++ | + | ± | - | - |
| | 32 | ++ | ++ | ++ | + | ± | - | - | - |
| | 64 | ++ | ++ | ++ | + | ± | - | - | - |
| | 128 | ++ | + | ± | - | - | - | - | - |

EXAMPLE 3

(1) Preparation of Sensitized Particles

Polyacrylamide particles "Affi-Gel 701" (manufactured by Bio-Rad Laboratories) were coloured by using Bismarck brown, and 1 g of the coloured particles was suspended in 19 ml of pH 7.2 PBS. The viral antigen suspension of rubella virus was prepared in the same manner as Example 1, and 20 ml of the viral antigen suspension were added to the above suspension of polyacrylamide particles. The mixture was kept at 4°C for 1 hour.

20 mg of l-ethyl-3[3-dimethylaminopropyl] carbodiimide.HCl (EDAC, manufactured by Bio-Rad Laboratories) were added to the above mixture, stirred well, and reacted at $4^{\circ}$C for 3 hours. After the reaction, the particles were washed 10 times with a saline solution by means of centrifugation, and suspended in 0.005M phosphate buffer solution of pH 7.2 containing 1% bovine serum albumin so that the concentration of the particles was 1%.

(2) Detection of Antivirus Antibody

The particles sensitized with rubella virus antigen were reacted with sera of rubella patients by the micro titre method.   the results obtained are shown in Table 4.

### T a b l e   4

#### Dilution Ratio of Serum

| Serum | 4 | 8 | 16 | 32 | 64 | 128 | 256 | 512 | HI Anti-body titre |
|---|---|---|---|---|---|---|---|---|---|
| 1 | ++ | ++ | + | ± | - | - | - | - | 32 |
| 2 | ++ | ++ | ++ | + | ± | - | - | - | 64 |
| 3 | ++ | ++ | ++ | ++ | + | ± | - | - | 128 |

### EXAMPLE 4

(1) Preparation of Sensitized Particles

Polyacrylamide particles, "Alfa-Gel 701", were coloured using direct blue, and 1 g of the coloured particles was suspended in 19 ml of pH 7.2 PBS.

A lyophilised HA antigen of measles virus (titre 1:32, manufactured by Denka Seiken Co.Ltd., Japan) was reconstituted with 1 ml of distilled water, and diluted 32 times with pH 7.2 PBS. The same procedure as in Example 3 was then carried out using 20 ml of this viral antigen solution and the above suspension of the particles, and a 1% suspension of the particles sensitized with rubella virus antigen was obtained.

(2) Detection of Antivirus Antibody

The sensitized particles were reacted with anti-measles virus anti-serum (titre 1:128, manufactured by Denka Seiken Co.Ltd., Japan) by the micro titre method, and the following result was obtained.

Table 5

Dilution Ratio of Serum

| 4 | 8 | 16 | 32 | 64 | 128 | 256 |
|---|---|----|----|----|-----|-----|
| ++ | ++ | ++ | ++ | + | ± | - |

EXAMPLE 5

(1) Preparation of Sensitized Particles

Carrier particles produced in step (1) of Example 1 were treated with PBS solution of pH 7.2 containing 1 ppm tannic acid in the same manner as in Example 2. The tannic acid-treated particles were sensitized with parainfluenza type-1 virus by using a lyophilised HA antigen of the same virus (titre 1:64, manufactured by Denka Seiken Co.Ltd., Japan) in the same manner as in Example 2. The sensitized particles were washed 4 times with a saline solution using centrifugation, and suspended in a dispersing medium so that the concentration of the particles was 1%.

(2) Detection of Antivirus Antibody

The particles sensitized with parainfluenza type-1 virus antigen thus obtained were reacted with anti-parainfluenza type-1 virus anti-serum (titre 1:128, manufactured by Denka Seiken Co.Ltd., Japan) by the micro titre method. The results obtained are shown in Table 6.

### T a b l e   6

| Dilution Ratio | Dilution Ratio of Serum | | | | | | | |
|---|---|---|---|---|---|---|---|---|
| of Antigen | 4 | 8 | 16 | 32 | 64 | 128 | 256 | 512 |
| 8 | ++ | ++ | ++ | ++ | ++ | ++ | ++ | ++ |
| 16 | ++ | ++ | ++ | ++ | ++ | ++ | ++ | ++ |
| 32 | ++ | ++ | ++ | ++ | ++ | ++ | + | ± |
| 64 | ++ | ++ | ++ | ++ | ++ | + | ± | - |
| 128 | ++ | + | ± | - | - | - | - | - |

### EXAMPLE 6

(1)  Preparation of Sensitized Particles

Polyacrylamide particles "Affi-Gel 701" were coloured using Bismarck brown, and the coloured particles were suspended in PBS of pH 7.2 in a concentration of 5%. 10 ml of this suspension of particles were mixed with 10 ml of PBS solution of pH 7.2 containing 5 ppm of tannic acid, and reacted at 37°C for 10 minutes.  The reacted particles were washed 10 times with a saline solution using centrifugation, and suspended in PBS of pH 7.2 in a concentration of 5%.

A lyophilised HA antigen of parainfluenza type-1 virus (titre 1:64, manufactured by Denka Seiken Co.Ltd., Japan) was reconstituted by adding 1 ml of distilled water, and diluted 64 times with PBS of pH 7.2.  10 ml of this viral antigen solution were mixed with 10 ml of the above tannic acid-treated particulate suspension, and the mixture was warmed at 37°C for 40 minutes.  The particles thereby sensitized with the viral antigen were washed 10 times with a saline solution using centrifugation, and suspended in 0.005M phosphate buffer solution of pH 7.2 containing 1% bovine serum albumin in a concentration of 1%.

(2)  Detection of Antivirus Antibody

The particles sensitized with parainfluenza type-1 virus antigen were reacted with antiparainfluenza type-1 virus anti-serum (titre 1:128, manufactured by Denka Seiken Co.Ltd., Japan) by the micro titre method.  The results obtained are shown in Table 7.

T a b l e  7

Dilution Ratio of Serum

| 4 | 8 | 16 | 32 | 64 | 128 | 256 | 512 |
|---|---|----|----|----|-----|-----|-----|
| ++ | ++ | ++ | ++ | ++ | + | ± | - |

EXAMPLE 7

(1)  Preparation of Sensitized Particles

The carrier particles produced in step (1) of Example 1 were treated with PBS solution of pH 7.2 containing 5 ppm tannic acid in the same manner as in Example 2.

A lyophilised HA mumps virus antigen (titre 1:32, manufactured by Denka Seiken Co.Ltd., Japan) was reconstituted with 1 ml of distilled water, and diluted 8 times with pH 7.2 PBS.

5 ml of this viral antigen solution were mixed with 5 ml of suspension of the above-indicated tannic acid-treated particles, and the mixture was warmed at 37°C for 40 minutes.  The particles thereby sensitized with the viral antigen were washed 4 times with a saline solution, suspended in a disperse medium, and lyophilised.

(2)  Detection of Antivirus Antibody

The lyophilised particles were reconstituted using distilled water, and reacted with anti-mumps virus anti-serum (titre 1:128, manufactured by Denka Seiken Co.Ltd., Japan) by the micro titre method.  The results obtained are shown in Table 8.

<u>T a b l e   8</u>

Dilution Ratio of Serum

| 4 | 8 | 16 | 32 | 64 | 128 | 256 | 512 |
|---|---|----|----|----|-----|-----|-----|
| ++ | ++ | ++ | ++ | ++ | + | ± | – |

## EXAMPLE 8

(1) Preparation of Sensitized Particles

Polyacrylamide particles "Affi-Gel 701" were coloured by Bismarck brown, and 0.5 g of the coloured particles were suspended in 9.5 ml of pH 7.2 PBS.

A lyophilised HA mumps virus antigen (titre 1:32, manufactured by Denka Seiken Co.Ltd., Japan) was reconstituted with 1 ml of distilled water, and diluted 16 times with pH 7.2 PBS.

10 ml of this viral antigen solution were mixed with the above particulate suspension, and kept at $4^{\circ}$C for 1 hour. 10 mg of EDAC were added to this mixture and the mixture was stirred well, and reacted at $4^{\circ}$C for 3 hours. After the reaction, the particles were washed 10 times with a saline solution using centrifugation, and suspended in 0.005M phosphate buffer solution of pH 7.2 containing 1% bovine serum albumin so that the concentration of the particles was 1%.

(2) Detection of Antivirus Antibody

The mumps virus antigen sensitized particles thus obtained reacted with anti-mumps virus anti-serum (titre 1:128, manufactured by Denka Seiken Co.Ltd., Japan) by the micro titre method. The results obtained are shown in Table 9.

<u>T a b l e   9</u>

Dilution of Ratio of Serum

| 4 | 8 | 16 | 32 | 64 | 128 | 256 | 512 |
|---|---|----|----|----|-----|-----|-----|
| ++ | ++ | ++ | ++ | + | ± | – | – |

Claims:

1. A reagent for use in the detection of antivirus antibody which comprises carrier particles which have the property of not aggregating to any significant extent in the presence of a virus having hemagglutinating activity and which have a particle diameter in the range of from 0.5 to 20 μm and a viral antigen having hemagglutinating activity and which is sensitized on the carrier particles.

2. A reagent as claimed in claim 1, wherein the carrier particles have a particle size of from 1 to 10 μm.

3. A reagent as claimed in claim 1 or 2, wherein the particles are spherical.

4. A reagent as claimed in any one of the preceding claims wherein the particles are gelatin particles or polyacrylamide particles.

5. A reagent as claimed in claim 4, wherein the gelatin particles comprise gelatin, a water-soluble polysaccharide and an alkali metal metaphosphate and are insolubilised with respect to water by cross-linking treatment with an aldehyde.

6. A reagent as claimed in any one of the preceding claims, wherein the viral antigen is the viral antigen of rubella virus, measles virus, mumps virus, parainfluenza virus type-1 or parainfluenza virus type-2.

7. A reagent as claimed in any one of the preceding claims, wherein the viral antigen is covalently bonded to the carrier particles by sensitization carried out by the tannic acid method.

8. A reagent as claimed in any one of the preceding claims which is in lyophilised form.

9. A method of detecting antivirus antibody, which comprises suspending in a liquid suspension medium the sensitized particles of the reagent of any one of the preceding claims, mixing a dilution series of a test sample with said suspension and identifying the existence of the antibody in any test mixture thus produced by the agglutination of sensitized particles therein.

10. A method as claimed in claim 9, wherein the test sample is serum.